# EUROPEAN PATENT SPECIFICATION

(11) **EP 1 124 548 B1**
(45) Date of publication and mention of the grant of the patent: **07.12.2005**
(21) Application number: 99952559.5
(22) Date of filing: 13.10.1999
(51) Int. Cl.: A61K 31/205, A61K 31/505, A61K 31/195, A61K 31/35, A23L 1/305, A23L 1/302, A61P 9/00

(54) **COMPOSITIONS FOR THE TREATMENT AND PREVENTION OF CARDIOVASCULAR DISEASES**
ZUBEREITUNGEN FÜR DIE BEHANDLUNG UND VERHINDERUNG VON KARDIOVASKULÄREN ERKRANKUNGEN
COMPOSITIONS DE TRAITEMENT ET DE PREVENTION DE MALADIES CARDIO-VASCULAIRES

(30) Priority: 30.10.1998 US 106205 P
(43) Date of publication of application: 22.08.2001
(73) Proprietor: Merck Patent GmbH, 64293 Darmstadt (DE)
(72) Inventor: BUCHHOLZ, Herwig, D-60599 Frankfurt (DE); MEDUSKI, Jerzy, D., Playa Del Rey, CA 90293-7640 (US)
(86) International application number: PCT/EP1999/007689
(87) International publication number: WO 2000/025764

(56) References cited:
- EP-A- 0 347 864
- WO-A-98/18491
- WO-A-98/33494
- WO-A-98/50051
- FR-A- 2 583 640
- US-A- 5 976 568
- DUELL P B ET AL: "HOMOCYST(E)INE: AN IMPORTANT RISK FACTOR FOR ATHEROSCLEROTIC VASCULAR DISEASE" CURRENT OPINION IN LIPIDOLOGY,GB,LONDON, vol. 8, no. 1, February 1997 (1997-02), pages 28-34, XP000853544 ISSN: 0957-9672

## Description

The invention relates to compositions to be used in the treatment and prevention of transmethylation disorders, preferably cardiovascular diseases such as atherogenic and thrombogenic diseases.

It has been documented that symptoms of cardiovascular diseases are often associated with elevated plasma levels of homocysteine (hyperhomocysteinemia). Investigations support the hypothesis that plasma homocysteine is a risk factor for coronary, cerebral, and peripheral occlusive diseases, as well as for carotid thickening. Furthermore, results of several studies show that the plasma homocysteine level is influenced by heredity. The data also suggest that there is no threshold effect but that a graded relation between elevated plasma levels of homocysteine and risk of cardiovascular disease exists (M.R. Malinow, J. Nutr. 126 (1996) 1238-1243).

In the human body, homocysteine is formed from methionine. In a simplified description methionine is condensed with adenosine triphosphate to produce S-adenosylmethionine and the latter is converted to S-adenosylhomocysteine. S-Adenosylhomocysteine is rapidly metabolised to homocysteine which is an important branch-point metabolite. It can be regenerated back to methionine, it can be converted to S-adenosylhomocysteine or it can enter the trans-sulfuration-pathway by reaction to cystathionine.

In the past, it has been the aim of several studies to treat hyperhomocysteinemia, i.e. to lower the elevated plasma homocysteine levels. It is known that betaine and choline, which is converted to betaine by oxidation, may act as methylating agents. For example, the enzyme-catalyzed reaction between betaine and homocysteine in the human organism leads to the formation of methionine and dimethylglycine and the treatment with betaine is known to be efficient in lowering homocysteine concentrations. Other attempts included the supplementation of vitamins like vitamin B₆, vitamin B₁₂ or usual multivitamins, vitamin B₆ in combination with a methionine restriction, methylcobalamine, folic acid, folic acid together with vitamins B₆ and B₁₂, folate, and occasionally folate in combination with vitamin B₆, vitamin B₁₂, choline or betaine (M.R. Malinow, J. Nutr. 126 (1996) 1238-1243).

However, the effectiveness of the previously applied compositions was not satisfactory. The treatment approach has not been linked to the complexity of the transmethylation metabolism.

The invention had the object of providing new compositions for the treatment and prevention of transmethylation disorders, preferably cardiovascular diseases such as atherogenic and thrombogenic diseases, which are more effective.

It has now been found that this object can be achieved with compositions which comprise one or more active ingredients and, optionally, one or more nutritional substances, solid, liquid and/or semiliquid excipients or auxiliaries, characterized in that the active ingredients consist of
a) a component A consisting of one or more compounds selected from methyl and methylene donors,
b) a component B consisting of 5-methyltetrahydrofolic acid or a physiologically acceptable salt thereof, and
c) a component C consisting of one or more bioflavonoids.

The invention furthermore relates to compositions to be used in the treatment and prevention of diseases associated with hyperhomocysteinemia.

Bioflavonoids are widely distributed among plants. High concentrations can be obtained from all citrus fruits, rose hips, and black currants. Commercial methods extract the rinds of oranges, tangerines, lemons, kumquats, and grapefruits. The bioflavonoids are known to strengthen the capillaries and to prevent oxidative damages in cells and tissues.

As mentioned above, it has been documented that cardiovascular diseases are associated with hyperhomocysteinemia. If homocysteine accumulates in unnormally high concentrations in the human body, e.g. in plasma, it is an indicator of atherogenic and thrombogenic conditions which lead to the formation of plaques in blood vessels and to the development of thrombi, i.e. to the coagulation of blood within blood vessels. According to the invention it has now been found that an elevated homocysteine level is a sign of the inadequate methyl pool involving both methyl donors and methyl transporters. Therefore, the homocysteine level in the human body may be used as an indicator of the state of the transmethylation metabolism in certain cases.

In the human body, one-carbon or C₁ groups exist in several oxidation states. These groups include methyl groups, methylene groups, methylidyne groups, carbonyl groups, formyl groups, hydroxymethyl groups, and carboxyl groups. Practically, these groups can be divided into groups at the oxidation level of methanol, formaldehyde, and formate. Exemplary sources of the methyl group (methanol oxidation level) are methionine, adenosylmethionine, methylated glycines, and choline. The sources of methylene group (formaldehyde oxidation level) are serine and glycine. One of the sources of the group at the formate oxidation level is e.g. histidine. All of these one-carbon groups form the so-called one-carbon pool and participate in many important reactions. As the methyl group is biochemically the most ubiquitous, the one-carbon pool is often referred to as the methyl pool.

The metabolic events of methyl groups are usually specified as transmethylation, whereas the involved molecules are called transmethylators. Depending upon their function during the metabolic events, the transmethylators are classified as methyl donors, methyl transporters, and methyl acceptors. Methyl donors are e.g. methionine, S-adenosylmethionine, choline, methylglycine (sarcosine), dimethylglycine, and trimethylglycine (betaine). Methyl transporters are e.g. tetrahydrofolates which are derived from folic acid and methylcobalamine and adenosyl-cobalamine, which are coenzymes derived from vitamin B₁₂. Methyl acceptors include all nucleic acids, proteins, most of them enzyme proteins, phospholipids (components of biomembranes), and many biological amines, which serve as neurotransmitters in many cases.

The properly functioning methylation of these four classes of acceptor molecules is of importance for their biochemical activity. The methylation of the nucleic acids assures their structure stability and their accurate genetic performance. The methylation of the enzyme proteins ensures their specificity and efficiency and prevents the accumulation of intermediary metabolites. The methylation of the phospholipids provides an optimal cytomembrane functionality and the methylation of the biological amines guarantees their specificity and effectiveness.

A decrease of the pool of methyl donors and/or of methyl transporters may lead to transmethylation disorders. Impairments of the methylation (also referred to as demethylations) of one or more components of the four methyl acceptor classes may occur and dysfunctions of these methyl acceptors may be the consequence. Metabolic dysfunctions and diseases may result.

Methyl acceptors also include many intermediary metabolites such as homocysteine. The methylation of homocysteine removes its negative effects on the vascular endothelia.

It has been documented that the major sites of atherosclerosis among humans with hyperhomocysteinemia are abdominal aorta, iliac arteries, internal carotid arteries, subclavian arteries, renal arteries, celiac trunk, mesenteric arteries, middle cerebral artery, basilar artery, and anterior cerebral arteries.

Major cardiovascular diseases associated with hyperhomocysteinemia are premature occlusive arterial disease, severe vascular disease in infancy and childhood, progressive arterial stenosis, intermittent claudication, renovascular hypertension, ischemic cerebrovascular disease, premature retinal artery and retinal vein occlusion, cerebral occlusive arterial disease, occlusive peripheral arterial disease, premature death due to thromboembolic disease, and ischemic heart disease (S.H. Mudd et al., Am. J. Hum. Genet. 33 (1981) 883-893; G.H.J. Boers et al., The New England Journal of Medicine 313(12) (1985) 709-715; R. Clarke et al., The New England Journal of Medicine 324(17) (1991) 1149-1155; S.S. Kang et al., Am. J. Hum. Genet. 48 (1991) 536-545; E.M. Wenzler et al., Am. J. Ophthalmol. 115(2) (1993) 162-167; R.P. Reis et al., Acta Med. Port. 7(5) (1994) 285-289; K. Robinson et al., Cleve. Clin. J. Med. 61 (6) (1994) 438-450; M.R. Malinow, J. Nutr. 126 (1996) 1238-1243; J.B. Ubbink et al., J. Nutr. 126 (1996) 1254-1257; M.R. Malinow et al., Arteriosclerosis, Thrombosis and Vascular Biology 17(6) (1997) 1157-1162; S.E.S. Miner et al., Clinical Chemistry 30(3) (1997) 189-201).

The invention furthermore relates to compositions to be used in the treatment and prevention of these major cardiovascular diseases.

The expression "methyl donor" (component A) stands for substances which are able to deliver methyl groups to transporter molecules.

The expression "methylene donor" (component A) stands for substances which are able to deliver methylene groups to transporter molecules.

The expression "methyl transporter" is used for substances which are able to transfer methyl groups to acceptor molecules. Therefore, the methyl transporters have to contain a transferable methyl group or they have to be able to remove a transferable methyl group from the donor molecules. Alternatively, they have to be able to remove another group from the donor molecules, but a group that may be converted to a transferable methyl group during the metabolic events or they have to contain such a group. For example, it is known that tetrahydrofolate may be converted to the 5-methyl derivative, which is able to transfer its methyl group to acceptor molecules. Furthermore, it is documented that the 5-methyl, 5-formyl, 10-formyl, 5,10-methylene, and 5,10-methenyl derivatives of tetrahydrofolate may be converted enzymatically into each other, i.e. each of these compounds may be converted to the 5-methyl derivative. Therefore, all of the above mentioned derivatives of tetrahydrofolate are methyl transporters within the meaning of the present invention.

If component A of the inventive compositions consists only of methylene donors, component B has to comprise the methyl transporter 5-methyl tetrahydrofolatic acid or a physiologically acceptable salt thereof which is able to remove methylene groups from the methylene donors and convert these methylene groups to transferable methyl groups.

Preferred methyl or methylene donors (component A) are selected from betaine, dimethylglycine, sarcosine, serine, and their physiologically acceptable salts.

The methylene donor serine is a component of phosphatidylserine, a naturally occurring phospholipid component of cellular membranes involved in a number of vital processes, such as nerve cell differentiation, activation and renewal, nerve transmitter production, ion transport etc.. The methylene donors may also function as methyl group suppliers.

Component B consists of 5-methyltetrahydrofolic acid (5-methyl-(6S)-tetrahydrofolic acid) or a physiologically acceptable salt thereof. L-5-methyltetrahydrofolic acid penetrates all organs of the human body.

The physiologically acceptable salts of the methyl donors and methylene donors can be obtained by converting a base of these compounds with an acid into the associated acid addition salt. Acids which yield physiologically harmless salts are e.g. inorganic acids, for example sulfuric acid, nitric acid, hydrochloric acid, phosphoric acids, such as orthophosphoric acid, organic acids, in particular aliphatic, alicyclic, araliphatic, aromatic or heterocyclic monobasic or polybasic carboxylic or sulfuric acids, for example formic acid, acetic acid, propionic acid, diethylacetic acid, malonic acid, succinic acid, fumaric acid, maleic acid, lactic acid, tartaric acid, malic acid, citric acid, gluconic acid, ascorbic acid or nicotinic acid.

Furthermore, the physiologically acceptable salts of the methyl donors and methylene donors can be obtained by converting an acid of these compounds with a base into one of its physiologically harmless metal salts or ammonium salts. Suitable salts in this context are, in particular, the sodium, potassium, magnesium, calcium and ammonium salts, and also substituted ammonium salts, for example the dimethyl-, diethyl- or diisopropylammonium salts, monoethanol-, diethanol- or diisopropylammonium salts, cyclohexyl- or dicyclohexylammonium salts or dibenzylethylenediammonium salts, and also, for example, salts with arginine or lysine.

The physiologically acceptable salts of the methyl transporter 5-methyltetrahydrofolic acid are selected from alkali metal or alkaline earth metal salts, preferably from sodium, potassium, magnesium, and calcium salts.

Preferred bioflavonoids (component C) are selected from mono-, di- or triglycoside bioflavonoids containing the aglycone quercetin. Especially preferred bioflavonoids are selected from isoquercetin (quercetin-3-glucoside; pyranoside form), quercitrin (quercetin-3-rhamnoside), isoquercitrin (quercetin-3-glucoside; furanoside form), quercimeritrin (quercetin-7-glucoside), spiraeosid (quercetin-4'-glucoside), rutin (quercetin-3-rutinoside), and hyperin (quercetin-3-galactoside).

If amino acids mentioned above or below may occur in more than one enantiomeric form, all of these forms and also mixtures thereof are included (e.g. the DL-forms). Preferably, the amino acids mentioned have (S)- or (L)-configuration even if this is not stated explicitly.

In preferred compositions according to the invention the molar ratio of component A : component B : component C is from 20,000: 1 : 10,000 to 500 : 1 : 100.

The present invention relates to compositions containing or comprising one or more active ingredients according to claim 1.

For instance, the present invention relates to food or food supplement compositions comprising one or more active ingredients according to claim 1. These food or food supplement compositions may optionally also comprise one or more solid, liquid and/or semiliquid excipients or auxiliaries which are known from prior art. The inventive food compositions comprise one or more active ingredients and one or more nutritional substances. The inventive food supplement compositions do not need to contain nutritional substances, but can be used for the preparation of food compositions.

The nutritional substances encompass all materials which are suited for consumption both by animals and/or by human beings, e.g. vitamins and provitamins thereof, fats, minerals or amino acids. Nutritional substances, which can be part of the inventive food compositions are e.g. materials, which are derived substantially from a single natural source such as sugar, unsweetened juice, nectar or puree from a single species of plant, such as unsweetened apple juice (including a blend of different varieties of apple juice), grapefruit juice, orange juice, apple sauce, apricot nectar, tomato juice, tomato sauce, tomato puree, grain plants of a single species and materials produced from grain plants of a single species, such as corn syrup, rye flour, wheat flour or oat bran. The inventive food compositions also comprise nutritional substances which are mixtures of different of the abovementioned materials, such as multivitamin preparations or sweetened juice. Further nutritional substances, which can be part of the inventive food compositions are e.g. food preparations such as breakfast foods, e.g. prepared cereals, toaster pastries, and breakfast drink mixes, infant formulas, dietary supplements, complete diet formulas, and weight-loss preparations, such as weight-loss drinks and weight-loss bars. Further examples for nutritional substances, which can be part of the inventive food compositions are e.g. animal feed or animal feed supplements (for example for poultry), and pet foods.

The nutritional substances include all edible combinations of carbohydrates, lipids, proteins, inorganic elements, trace elements, vitamins, water, and active metabolites of plants and animals.

The inventive food or food supplement compositions are preferably used for oral administration, e.g. in the form of food, pills, tablets, capsules, powders, syrups, solutions, or suspensions or as products absorbable through mucosal membranes.

The inventive food or food supplement compositions can be prepared by methods which are well-known to the expert.

Furthermore, the present invention relates to pharmaceutical compositions comprising one or more active ingredients according to claim 1. These inventive pharmaceutical compositions can be used in human or veterinary medicine. Suitable excipients are organic or inorganic substances which are suitable for enteral, parenteral or topical administration and which do not react with the novel compounds, for example water, vegetable oils, benzyl alcohols, alkylene glycols, polyethylene glycols, glycerol triacetate, gelatin, carbohydrates such as lactose or starch, magnesium stearate, talc, petrolatum. Used for oral administration are, in particular, tablets, pills, coated tablets, capsules, powders, granules, syrups, solutions or drops, for rectal administration are suppositories, for parenteral administration are solutions, - preferably oily or aqueous solutions, also suspensions, emulsions or implants, for topical administration are ointments, creams or powders. The novel pharmaceutical compositions can also be lyophilized, and the resulting lyophilizates be used, for example, for producing injection preparations. The stated preparations can be sterilized and/or comprise auxiliaries such as lubricants, preservatives, stabilizers and/or wetting agents, emulsifiers, salts to influence the osmotic pressure, buffer substances, colorants, flavourings and/or several other active substances, for example one or more vitamins.

The specific dose for each patient depends on a wide variety of factors, for example on the activity of the specific compounds employed, on the age, bodyweight, general state of health, sex, on the diet, the time and route of administration, on the rate of excretion, medicinal substance combination and severity of the particular disorder for which the therapy is applied. Oral administration is preferred.

The invention furthermore relates to the use of one or more compounds selected from methyl and methylene donors, 5-methyltetrahydrofolic acid or a physiologically acceptable salt thereof and one or more bioflavonoids for the preparation of compositions (e.g. pharmaceutical, food or food supplement compositions) for the treatment of transmethylation disorders, in particular by non-chemical means. The methyl donors, methylene donors, methyl transporters, and bioflavonoids can for this purpose be converted into a suitable dosage form, optionally together with one or more nutritional substances, solid, liquid and/or semiliquid excipients or auxiliaries and, where appropriate, in combination with one or more other active substances.

The inventive compositions can be used for controlling transmethylation disorders, in particular cardiovascular diseases such as atherogenic and thrombogenic diseases.

The compounds used in the inventive compositions are commercially available or may be prepared in accordance with methods known per se and as described in the literature (for example in the standard works such as Houben-Weyl, Methoden der organischen Chemie [Methods of Organic Chemistry], Georg-Thieme-Verlag, Stuttgart).

From the foregoing description, one skilled in the art can easily ascertain the essential characteristics of this invention and, without departing from the spirit and scope thereof, can make various changes and modifications of the invention to adapt it to various usages and conditions. Therefore, the following examples should be interpreted as an illustration of the present invention but not as a limitation thereof.

The following example 1 relates to the composition of active ingredients for one single serving.

### Example 1

The active ingredients of the composition consist of

| | |
|---|---|
| Betaine anhydrous | 600 mg |
| L-5-Methyltetrahydrofolic acid, calcium salt | 0.5 mg |
| Isoquercetin | 500 mg |

and are related to an average bodyweight of about 70 kg.

The following example A relates to a pharmaceutical preparation:

### Example A: Tablets

A mixture of 1 kg of the composition of active ingredients as given in example 1 and the appropriate amount of excipients (4 kg of lactose, 1.2 kg of potato starch, 0.2 kg of talc, and 0.1 kg of magnesium stearate) is compressed to tablets in a customary way so that each tablet comprises 1000 mg of the composition of active ingredients as given in example 1.

## Claims

1. Composition comprising one or more active ingredients and, optionally, one or more nutritional substances, solid, liquid and/or semiliquid excipients or auxiliaries, **characterized in that** the active ingredients consist of
a) a component A consisting of one or more compounds selected from methyl and methylene donors,
b) a component B consisting of 5-methyltetrahydrofolic acid or a physiologically acceptable salt thereof, and
c) a component C consisting of one or more bioflavonoids.

2. Composition according to claim 1, **characterized in that** component A consists of one or more compounds selected from betaine, dimethylglycine, sarcosine and serine or their physiologically acceptable salts.

3. Composition according to one of claims 1 or 2, **characterized in that** component C consists of one or more compounds selected from mono-, di- or triglycoside bioflavonoids containing the aglycone quercetin.

4. Composition according to claim 3, **characterized in that** component C consists of one or more compounds selected from isoquercetin, quercitrin, isoquercitrin, quercimeritrin, spiraeosid, rutin, and hyperin.

5. Use of one or more compounds selected from methyl and methylene donors, 5-methyltetrahydrofolic acid or a physiologically acceptable salt thereof, and one or more bioflavonoids in the preparation of a composition for the treatment and prevention of transmethylation disorders.

6. Use of one or more compounds selected from methyl and methylene donors, 5-methyltetrahydrofolic acid or a physiologically acceptable salt thereof, and one or more bioflavonoids in the preparation of a composition for the treatment and prevention of cardiovascular diseases.

7. Use of one or more compounds selected from methyl and methylene donors, 5-methyltetrahydrofolic acid or a physiologically acceptable salt thereof, and one or more bioflavonoids in the preparation of a composition for the treatment and prevention of atherogenic and/or thrombogenic diseases.

8. Use of one or more compounds selected from methyl and methylene donors, 5-methyltetrahydrofolic acid or a physiologically acceptable salt thereof, and one or more bioflavonoids in the preparation of a composition for the treatment and prevention of diseases associated with hyperhomocysteinemia.

9. Use of one or more compounds selected from methyl and methylene donors, 5-methyltetrahydrofolic acid or a physiologically acceptable salt thereof, and one or more bioflavonoids in the preparation of a composition for the treatment and prevention of premature occlusive arterial disease, severe vascular disease in infancy and childhood, progressive arterial stenosis, intermittent claudication, renovascular hypertension, ischemic cerebrovascular disease, premature retinal artery and retinal vein occlusion, cerebral occlusive arterial disease, occlusive peripheral arterial disease, premature death due to thromboembolic disease and/or ischemic heart disease.

## Patentansprüche

1. Zusammensetzung enthaltend einen oder mehrere Wirkstoffe und gegebenenfalls einen oder mehrere Nährstoffe, feste, flüssige und/oder halbflüssige Träger oder Hilfsstoffe, **dadurch gekennzeichnet, dass** die Wirkstoffe bestehen aus:
a) einer Komponente A bestehend aus einer oder mehreren aus Methyl- und Methylen-Donatoren ausgewählten Verbindungen,
b) einer Komponente B bestehend aus 5-Methyltetrahydrofolsäure oder einem pharmazeutisch verträglichen Salz davon, und
c) einer Komponente C bestehend aus einem oder mehreren Bioflavonoiden.

2. Zusammensetzung nach Anspruch 1, **dadurch gekennzeichnet, dass** die Komponente A aus einer oder mehreren Verbindungen ausgewählt aus Betain, Dimethylglycin, Sarkosin und Serin oder deren pharmazeutisch verträglichen Salzen besteht.

3. Zusammensetzung nach einem der Ansprüche 1 oder 2, **dadurch gekennzeichnet, dass** die Komponente C aus einer oder mehreren Verbindungen ausgewählt aus Mono-, Di-, oder Triglycosid-Bioflavonoiden, die das Aglykon Quercetin enthalten, besteht.

4. Zusammensetzung nach Anspruch 3, **dadurch gekennzeichnet, dass** die Komponente C aus einer oder mehreren Verbindungen ausgewählt aus Isoquercetin, Quercitrin, Isoquercitrin, Quercimeritrin, Spiraeosid, Rutin und Hyperin besteht.

5. Verwendung von einer oder mehreren Verbindungen, ausgewählt aus Methyl- und Methylen-Donatoren, 5-Methyltetrahydrofolsäure oder einem pharmazeutisch verträglichen Salz davon, und einem oder mehreren Bioflavonoiden, bei der Herstellung einer Zusammensetzung zur Behandlung und Vorbeugung von Transmethylierungs-Störungen.

6. Verwendung von einer oder mehreren Verbindungen, ausgewählt aus Methyl- und Methylen-Donatoren, 5-Methyltetrahydrofolsäure oder einem pharmazeutisch verträglichen Salz davon, und einem oder mehreren Bioflavonoiden, bei der Herstellung einer Zusammensetzung zur Behandlung und Vorbeugung von kardiovaskulären Erkrankungen.

7. Verwendung von einer oder mehreren Verbindungen, ausgewählt aus Methyl- und Methylen-Donatoren, 5-Methyltetrahydrofolsäure oder einem pharmazeutisch verträglichen Salz davon, und einem oder mehreren Bioflavonoiden bei der Herstellung einer Zusammensetzung zur Behandlung und Vorbeugung von atherogenen und/oder thrombogenen Erkrankungen.

8. Verwendung von einer oder mehreren Verbindungen, ausgewählt aus Methyl- und Methylen-Donatoren, 5-Methyltetrahydrofolsäure oder einem pharmazeutisch verträglichen Salz davon, und einem oder mehreren Bioflavonoiden, bei der Herstellung einer Zusammensetzung zur Behandlung und Vorbeugung von Erkrankungen, die mit Hyperhomocysteinämie einhergehen.

9. Verwendung von einer oder mehreren Verbindungen, ausgewählt aus Methyl- und Methylen-Donatoren, 5-Methyltetrahydrofolsäure oder einem pharmazeutisch verträglichen Salz davon, und einem oder mehreren Bioflavonoiden, bei der Herstellung einer Zusammensetzung zur Behandlung und Vorbeugung von vorzeitiger Arterienverschlusserkrankung, schwerer Gefäßerkrankung im Säuglingsalter und in der Kindheit, fortschreitender Arterienstenose, Claudicatio intermittens, renovaskulärer Hypertonie, ischämischer cerebrovaskulärer Erkrankung, vorzeitigem Verschluss der Netzhautarterie und der Netzhautvene, cerebraler Arterienverschlusserkrankung, peripherer Arterienverschlusserkrankung, vorzeitigem Tod aufgrund von Thromboembolie-Erkrankung und/oder ischämischer Herzerkrankung.

## Revendications

1. Composition comprenant un ou plusieurs ingrédients actifs et, en option, une ou plusieurs substances nutritionnelles, un ou plusieurs excipients ou auxiliaires solides, liquides et/ou semi-liquides, **caractérisée en ce que** les ingrédients actifs sont constitués par :
a) un composant A qui est constitué par un ou plusieurs composés choisis parmi des donneurs de méthyle et de méthylène ;
b) un composant B qui est constitué par un acide 5-méthyltétrahydrofolique ou par un sel afférent acceptable physiologiquement ;et
c) un composant C qui est constitué par un ou plusieurs bioflavonoïdes.

2. Composition selon la revendication 1, **caractérisée en ce que** le composant A est constitué par un ou plusieurs composés choisis parmi bétaïne, diméthylglycine, sarcosine et sérine ou leurs sels physiologiquement acceptables.

3. Composition selon l'une des revendications 1 ou 2, **caractérisée en ce que** le composant C est constitué par un ou plusieurs composés choisis parmi des bioflavonoïdes mono-, di- ou triglycosides contenant de la quercétine aglycone.

4. Composition selon la revendication 3, **caractérisée en ce que** le composant C est constitué par un ou plusieurs composés choisis parmi isoquercétine, quercétine, isoquercitrine, querciméritrine, spiraéoside, rutine et hypérine.

5. Utilisation d'un ou de plusieurs composés choisis parmi des donneurs de méthyle et de méthylène, acide 5-méthyltétrahydrofolique ou un sel afférent acceptable physiologiquement, et un ou plusieurs bioflavonoïdes au niveau de la préparation d'une composition pour le traitement et la prévention de troubles de transméthylation.

6. Utilisation d'un ou de plusieurs composés choisis parmi des donneurs de méthyle et de méthylène, acide 5-méthyltétrahydrofolique ou un sel afférent acceptable physiologiquement, et un ou plusieurs bioflavonoïdes au niveau de la préparation d'une composition pour le traitement et la prévention de maladies cardiovasculaires.

7. Utilisation d'un ou de plusieurs composés choisis parmi des donneurs de méthyle et de méthylène, acide 5-méthyltétrahydrofolique ou un sel afférent acceptable physiologiquement, et un ou plusieurs bioflavonoïdes au niveau de la préparation d'une composition pour le traitement et la prévention de maladies athérogéniques et/ou thrombogéniques.

8. Utilisation d'un ou de plusieurs composés choisis parmi des donneurs de méthyle et de méthylène, acide 5-méthyltétrahydrofolique ou un sel afférent acceptable physiologiquement, et un ou plusieurs bioflavonoïdes au niveau de la préparation d'une composition pour le traitement et la prévention de maladies qui sont associées à l'hyperhomocystéinémie.

9. Utilisation d'un ou de plusieurs composés choisis parmi des donneurs de méthyle et de méthylène, acide 5-méthyltétrahydrofolique ou un sel afférent acceptable physiologiquement, et un ou plusieurs bioflavonoïdes au niveau de la préparation d'une composition pour le traitement et la prévention d'une maladie artérielle occlusive prématurée, d'une maladie vasculaire sévère dans la petite enfance et l'enfance, d'une sténose artérielle progressive, d'une claudication intermittente, d'une hypertension rénovasculaire, d'une maladie cérébrovasculaire ischémique, d'une occlusion prématurée d'artère rétinienne et de veine rétinienne, d'une maladie artérielle d'occlusion cérébrale, d'une maladie artérielle périphérique occlusive, d'une mort prématurée due à une maladie thrombo-embolique et/ou une maladie cardiaque ischémique.
